# EUROPEAN PATENT SPECIFICATION

(11) **EP 0 265 932 B1**
(45) Date of publication and mention of the grant of the patent: **20.01.1993**
(21) Application number: 87115837.4
(22) Date of filing: 28.10.1987
(51) Int. Cl.: C07C 209/68

(54) **Process for the preparation of alylanaline using an aluminosilicate catalyst**
Verfahren zur Herstellung von Alkylanilin mit einem Aluminosilikat-Katalysator
Procédé pour la préparation d'alkylaniline avec un catalyseur aluminosilicate

(30) Priority: 31.10.1986 US 925335
(43) Date of publication of application: 04.05.1988
(73) Proprietor: CHEVRON RESEARCH AND TECHNOLOGY COMPANY, San Francisco, California 94105 (US)
(72) Inventor: Bacskai, Robert, Kensington, CA 94708 (US); Valeiras, Horacio A., Alameda, CA 94501 (US)
(74) Representative: Müller-Boré & Partner Patentanwälte

(56) References cited:
- EP-A- 0 226 781
- DE-A- 2 111 194
- FR-A- 1 406 739
- GB-A- 863 539
- US-A- 3 923 892

## Description

The present invention relates to a process for the preparation of an alkylaniline having about 10 to 30 carbon atoms in the alkyl substituent which comprises reacting aniline with an olefin in the presence of an aluminosilicate clay catalyst.

It is known in the art that aromatic amines, such as aniline, may be alkylated with lower olefins in the presence of aluminosilicate catalysts. In general, the olefins employed to alkylate the aromatic amine have been lower molecular weight olefins of up to 5 carbon atoms, such as ethylene, propylene, butylene, isobutylene and amylene.

For example, an article by P. B. Venuto et al. in the Journal of Catalysis, Vol. 5, pp. 81-98 (1966) describes the alkylation of aromatic compounds with a variety of alkylating agents, such as olefins, alcohols and haloalkanes, in the presence of a crystalline aluminosilicate catalyst. Among the reactions studied, this article describes the alkylation of aniline with propylene and ethylene using an acid zeolite catalyst. The zeolite catalyzed reaction of aniline with propylene is taught as forming mono and di-isopropylaniline, and the reaction of aniline with ethylene is taught as forming ortho and paraethylaniline and diethylaniline.

British Patent No. 846,226 (1960) discloses a process for the preparation of tertiary-butylphenylamines by reacting phenylamines with isobutylene in the presence of activated, substantially neutral bleaching earths of the montmorillonite type. The phenylamines which are contemplated for use in this process are aniline, toluidine and xylidine.

Chemical Abstracts, Vol. 62, 9040 (1965) describes the alkylation of aniline with lower olefins having up to 5 carbon atoms using an aluminosilicate catalyst. Specific olefins employed as alkylating agents are propylene, butylene, isobutylene and amylene.

FR-A-1 406 739 discloses a process for alkylating alkylatable cyclic compounds which comprises effecting ring alkylation of a polar-substituted aromatic compound or a heterocyclic compound in the presence of a catalyst comprising a crystalline aluminosilicate having exchangeable cations and an ordered internal structure, said ordered internal structure having a defined pore size of at least 6 Å. The aluminosilicate having exchangeable cations and an ordered internal structure preferably is faujasitic, particularly a sodium form or a rare earth exchanged form of faujasite, or a hydrogen exchange Zeolite Y. The alkylating agent can be an olefin having 2 to 20 carbon atoms. In one example the alkylation of aniline with propylene in the presence of Zeolite X having a pore size of 30 Å as catalyst is disclosed.

The present invention provides a process for the preparation of alkylaniline having from 10 to 30 carbon atoms in the alkyl substituent which comprises reacting aniline with an olefin having from 10 to 30 carbon atoms in the presence of an aluminosilicate clay catalyst.

Among other factors, the present invention is based on the surprising discovery that relatively long chain alkylanilines can be conveniently prepared in good yields by the reaction of aniline with higher molecular weight olefins having 10 to 30 carbon atoms in the presence of an aluminosilicate clay catalyst.

The process of the present invention will generally employ an olefin containing from 10 to 30 carbon atoms. Olefins which are suitable for reaction with aniline may be either straight chain, slightly branched or highly branched in structure. These olefins are normally obtained by conventional procedures known to the art. For example, straight chain olefins may be conveniently obtained by the cracking of wax or from the ethylene growth reaction. Branched chain olefins may be readily prepared by the polymerization of lower molecular weight olefins, such as propylene or isobutylene. Preferred olefins for reaction with aniline are those which contain 12 to 24 carbon atoms. Examples of preferred olefins include 1-dodecene and a (C₂₀₋₂₄)-alpha olefin.

According to the process of the invention, the reaction of aniline with an olefin is carried out in the presence of an aluminosilicate clay catalyst. The aluminosilicate clay catalyst may be any of the various aluminosilicate clays. In general, the aluminosilicates which are suitable for use in the present invention are those having a silica to alumina molar ratio in the range of 3:1 to 150:1, preferably in the range of 3:1 to 6:1. The amount of catalyst utilized will generally range from 0.1 weight percent to 20 weight percent.

The aluminosilicate catalyst may be any of the commercially available natural clays, which are generally hydrated aluminum silicates having a very fine particle size of irregularly shaped crystals. Typical clays which are useful as catalysts in the present process include kaolin, montmorillonite, attapulgite, illite, bentonite, halloysite and mullite. A particularly suitable clay catalyst is Filtrol Grade 22 clay, available from Filtrol Corp., which is an aluminosilicate clay characterized as a highly activated adsorbent used for the low temperature decolorization of animal, vegetable and mineral oils.

The molar ratio of olefin to aniline will normally range from 1:10 to 10:1, and preferably will range from 1:3 to 3:1.

The aluminosilicate catalyzed reaction of aniline with an olefin will generally take place in a pressure reactor at a pressure in the range of 2.76 bar (40 psi) to 34.45 bar (500 psi), preferably in the range of 5.52 bar (80 psi) to 24.1 bar (350 psi). The reaction temperature will generally range from 150°C to 350°C, preferably from 250°C to 320°C. The reaction will normally proceed over a period of about 0.5 to 8 hours. The resulting alkylamine is then separated from the catalyst residue and unreacted starting material, using conventional techniques.

The alkylaniline produced by the process of the present invention will generally contain a mixture of isomers, including ortho-, para-, and N-substituted alkylaniline. In general, the ortho-substituted alkylaniline will be the predominant isomer produced.

The alkyl side chain on the alkylaniline will normally contain from 10 to 30 carbon atoms, and preferably will contain from 12 to 24 carbon atoms. Examples of preferred alkylanilines produced by the present process include dodecylaniline and (C₂₀₋₂₄)-alkylaniline.

The alkylanilines prepared by the process of the invention have utility as intermediates for the preparation of oligomeric amines which are useful as corrosion inhibitors, as described in U.S. Patent No. 4,780,278 , filed concurrently herewith, entitled "Alkylaniline/Formaldehyde Oligomers as Corrosion Inhibitors", and U.S. Patent No. 4,778,654 , filed concurrently herewith, entitled "Alkylaniline/Formaldehyde Co-oligomers as Corrosion Inhibitors".

The following examples are provided to illustrate the invention in accordance with the principles of this invention but are not to be construed as limiting the invention in any way except as indicated by the appended claims.

### Example 1

### Preparation of Dodecylaniline from Aniline and 1-Dodecene

A one-liter stainless steel autoclave was charged with 333 g (3.57 M) aniline, 169 g (1.0 M) 1-dodecene and 47.7 g Filtrol Clay 22 catalyst. The autoclave was purged with nitrogen and was then heated at 300°C under nitrogen pressure (about 300 psi) and stirred for 2 hours. After cooling to room temperature, the reaction mixture was diluted with 300 ml toluene, washed with 400 ml of 5% NaOH and twice with 400 ml of water. Gas chromatographic analysis showed that the toluene solution contained 121.8 g (0.467 M) dodecylaniline. The toluene was evaporated and the residue was distilled under vacuum.

The boiling point of the dodecylaniline was found to be 169-178°C/120 Pa (0.9 mm Hg).

Nitrogen analysis of the dodecylaniline distillate showed 5.45% nitrogen (theory = 5.36%). ¹³C-NMR analysis showed the presence of 62% ortho-dodecylaniline, 10% para-dodecylaniline and 27% N-dodecylaniline.

### Example 2

### Preparation of (C₂₀₋₂₄)-Alkylaniline from Aniline and (C₂₀₋₂₄)-Alpha-Olefin

A one-liter stainless steel autoclave was charged with 171 g (1.84 M) aniline, 147 g (0.5 M) of a (C₂₀₋₂₄)-alpha olefin obtained by the ethylene growth reaction, and 30.3 g Filtrol Clay 22 catalyst. The autoclave was purged with nitrogen, then heated under nitrogen pressure (about 13.8 bar (200 psi)) at 300°C and stirred for 2 hours. After cooling to room temperature, the reaction mixture was diluted with 678 ml toluene, and washed with 300 ml of 5% NaOH. Gas chromatographic analysis showed that the toluene solution contained 77.6 g (0.2 M) of (C₂₀₋₂₄)-alkylaniline and 82.0 g (0.28 M) of (C₂₀₋₂₄)-alpha-olefin. The toluene was evaporated and the residue was distilled under vacuum. The fraction boiling at 230°C 80 Pa (0.6 mm Hg) was determined to be 98.3% (C₂₀₋₂₄)-alkylaniline.

Nitrogen analysis of the above distillate showed 3.6% nitrogen (theory = 3.65%). ¹³C-NMR analysis showed the presence of 70% ortho-substituted (C₂₀₋₂₄)-alkylaniline, 10% para-substituted (C₂₀₋₂₄)-alkylaniline and 20% N-substituted (C₂₀₋₂₄)-alkylaniline.

### Examples 3 and 4

### Preparation of Dodecylaniline

A 16 ml stainless steel shaker type microreactor was charged under nitrogen with 7.0 g (0.075 M) aniline, 3.5 g (0.021 M) 1-dodecene and 1.0 g of an aluminosilicate clay catalyst as indicated in Table I. The molar ratio of aniline to 1-dodecene was 3.6:1. The reaction mixture was shaken at 300°C for 2 hours. After cooling to room temperature, the reaction mixture was diluted with 20 ml toluene containing a 1-tetradecene internal standard. Following washing with 20 ml of 5% NaOH, the toluene solution was analyzed by gas chromatography. The results are summarized in Table I.

Following a similar procedure, 2.0 g (0.0215 M) aniline, 8.5 g (0.0503 M) 1-dodecene and 1.0 g of an aluminosilicate clay catalyst, as indicated in Table II, were reacted at 300°C for 2 hours. The molar ratio of 1-dodecene to aniline was 2.3:1. The results are summarized in Table II.

**Table I**

| Preparation of Dodecylaniline Using Aluminosilicate Catalyst^{a} | | | | |
|---|---|---|---|---|
| Ex. No. | Catalyst | Conversion, %^{b} | Selectivity | |
| | | | Mono-alkyl aniline, % | Dialkyl aniline, % |
| 3 | Filtrol Clay 22 | 43.4 | 67.8 | Trace |

| | | | | |
|---|---|---|---|---|
| ^{a}Molar ratio of aniline to 1-dodecene=3.6:1 | | | | |
| ^{b}Based on 1-dodecene | | | | |

**Table II**

| Preparation of Dodecylaniline Using Aluminosilicate Catalyst^{a} | | | | |
|---|---|---|---|---|
| Ex. No. | Catalyst | Conversion, %^{b} | Selectivity | |
| | | | Mono-alkyl aniline, % | Dialkyl aniline, % |
| 4 | Filtrol Clay 22 | 87.0 | 59.9 | 18.7 |

| | | | | |
|---|---|---|---|---|
| ^{a}Molar ratio of 1-dodecene to aniline = 2.3:1 | | | | |
| ^{b}Based on aniline | | | | |

### Example 5

### Preparation of (C₂₀₋₂₄)-Alkylaniline from Aniline and (C₂₀₋₂₄)-Alpha-Olefin

A one-gallon stainless steel autoclave was charged with 644 g (6.92 M) aniline, 1343 g (4.60 M) (C₂₀₋₂₄)-alpha-olefin and 198 g Filtrol Clay 22 catalyst. The autoclave was heated and stirred for 2 hours at 300°C under aniline vapor pressure (about 16.5 bar (240 psi)). After cooling to about 50°C, the mixture was pumped out of the autoclave and filtered to remove the catalyst. Gas chromatographic analysis showed that the reaction product mixture contained 523 g (1.35 M) of (C₂₀₋₂₄)-alkylaniline and 833 g (2.85 M) of (C₂₀₋₂₄)-alpha-olefin. The conversion of (C₂₀₋₂₄)-alpha-olefin was 38%. The selectivity to mono-alkylaniline was 77%.

## Claims

1. A process for the preparation of alkylaniline having from 10 to 30 carbon atoms in the alkyl substituent which comprises reacting aniline with an olefin having from 10 to 30 carbon atoms in the presence of an aluminosilicate catalyst,which aluminosilicate is a clay.

2. The process according to Claim 1, wherein the olefin has from 12 to 24 carbon atoms.

3. The process according to Claim 2, wherein the olefin is 1-dodecene.

4. The process according to Claim 2, wherein the olefin is a (C₂₀₋₂₄)-alpha-olefin.

5. The process according to Claim 1, wherein the molar ratio of olefin to aniline is 1:10 to 10:1.

6. The process according to Claim 1, wherein the reaction is carried out at a temperature of 150°C to 350°C.

7. The process according to Claim 1, wherein the reaction is carried out at a pressure of 2.76 bar (40 psi) to 34.45 bar (500 psi).

## Patentansprüche

1. Verfahren zur Herstellung von Alkylanilin mit 10 bis 30 Kohlenstoffatomen im Alkylsubstituenten, dadurch gekennzeichnet, daß man Anilin mit einem Olefin mit 10 bis 30 Kohlenstoffatomen in Gegenwart eines Aluminosilikat-Katalysators umsetzt, wobei das Aluminosilikat ein Ton ist.

2. Verfahren nach Anspruch 1, dadurch gekennzeichnet, daß das Olefin 12 bis 24 Kohlenstoffatome hat.

3. Verfahren nach Anspruch 2, dadurch gekennzeichnet, daß das Olefin 1-Dodecen ist.

4. Verfahren nach Anspruch 2, dadurch gekennzeichnet, daß das Olefin ein (C₂₀₋₄)-alpha-Olefin ist.

5. Verfahren nach Anspruch 1, dadurch gekennzeichnet, daß das molare Verhältnis von Olefin zu Anilin 1:10 bis 10:1 beträgt.

6. Verfahren nach Anspruch 1, dadurch gekennzeichnet, daß die Reaktion bei einer Temperatur von 150°C bis 350°C durchgeführt wird.

7. Verfahren nach Anspruch 1, dadurch gekennzeichnet, daß die Reaktion bei einem Druck von 2,76 bar (40 psi) bis 34,45 bar (500 psi) durchgeführt wird.

## Revendications

1. Procédé de production d'alkylaniline ayant 10 à 30 atomes de carbone dans le substituant alkyle, qui consiste à faire réagir de l'aniline avec une oléfine ayant 10 à 30 atomes de carbone en présence d'un catalyseur à base d'un aluminosilicate, cet aluminosilicate étant une argile.

2. Procédé suivant la revendication 1, dans lequel l'oléfine a 12 à 24 atomes de carbone.

3. Procédé suivant la revendication 2, dans lequel l'oléfine est le 1-dodécène.

4. Procédé suivant la revendication 2, dans lequel l'oléfine est une alpha-oléfine en C₂₀ à C₂₄.

5. Procédé suivant la revendication 1, dans lequel le rapport molaire de l'oléfine à l'aniline est de 1:10 à 10:1.

6. Procédé suivant la revendication 1, dans lequel la réaction est conduite à une température de 150°C à 350°C.

7. Procédé suivant la revendication 1, dans lequel la réaction est conduite à une pression de 2,76 bars (40 lb/in²) à 34,45 bars (500 lb/in²).
